# EUROPÄISCHE PATENTSCHRIFT

(11) **EP 1 743 673 B1**
(45) Veröffentlichungstag und Bekanntmachung des Hinweises auf die Patenterteilung: **23.04.2008**
(21) Anmeldenummer: 05015074.7
(22) Anmeldetag: 12.07.2005
(51) Int. Cl.: A61M 37/00

(54) **Antriebsvorrichtung für ein Gerät zum lokalen Aufstechen einer Haut und Verfahren zum Betreiben der Antriebsvorrichtung**
Drive means for an apparatus for perforating locally a skin and method to operate the apparatus
Dispositif de contrôle d'un appareil pour percer localement la peau et méthode pour le fonctionnement du dispositif

(43) Veröffentlichungstag der Anmeldung: 17.01.2007
(73) Patentinhaber: MT Derm GmbH, 14167 Berlin (DE)
(72) Erfinder: Kluge, Jörn, 14513 Teltow (DE)
(74) Vertreter: Bittner, Thomas L.

(56) Entgegenhaltungen:
- US-A- 4 671 277
- US-A- 4 782 725
- US-A- 5 054 339
- US-A- 5 471 102
- US-A- 5 976 167

## Beschreibung

Die Erfindung betrifft eine Antriebsvorrichtung für ein Gerät zum lokalen Aufstechen einer Haut, insbesondere zum Aufbringen von permanentem Make-up oder eines Tattoos, und ein Verfahren zum Betreiben der Antriebsvorrichtung.

### Stand der Technik

Geräte zum lokalen Aufstechen einer Haut werden beispielsweise zum Aufbringen von permanentem Make-up oder eines Tattoos auf menschliche oder tierische Haut genutzt und umfassen üblicherweise eine Antriebsvorrichtung mit Antriebsmitteln zum Erzeugen einer repetierenden Schubbewegung, die in einem an die Antriebsmittel gekoppelten Modul zum Ein- / Ausfahren von Aufstechmitteln genutzt wird, um die Haut aufzustechen, auf welche das permanente Make-up oder das Tattoo aufzubringen ist. Bei den Aufstechmitteln handelt es sich üblicherweise um eine Nadel oder ein Nadelsystem, welches mehrere Nadeln umfaßt. Nach dem Aufstechen der Haut kann eine entlang der Aufstechmittel zum Aufstechort geführte Farbe in die Hautoberfläche eindringen. Solche Geräte sind beispielsweise in dem Dokument US 6,345,553 beschrieben.

Beim Aufstechen der zu behandelnden Haut wird der Schubbewegung der Nadel / des Nadelsystems eine Widerstandskraft durch die Hautbestandteile entgegengesetzt. Diese Widerstandskraft hängt von den individuellen Eigenschaften der Haut im aufzustechenden Oberflächenbereich ab. Bei bekannten Geräten zum Aufbringen von permanentem Make-up oder eines Tattoos kann mit Hilfe eines Wahlschalters eine vorgegebene Repetier- oder Wiederholfrequenz der repetierenden / wiederholt ausgeführten Schubbewegung und somit der Häufigkeit des Hautaufstechens pro Zeiteinheit festgelegt werden, die unabhängig von der der repetierenden Schubbewegung entgegengesetzten Widerstandskraft konstant gehalten wird. Dieses geschieht, indem die an die Antriebsmittel angelegten Strom- / Spannungswerte zur Aufrechterhaltung einer konstanten Repetierfrequenz nachgeregelt werden, auch wenn eine geänderte Widerstandskraft infolge geänderter Hauteigenschaften auftritt. Wenn eine solches Konstanthalten der Repetierfrequenz nicht vorgesehen ist, kann es zum Absinken der Repetierfrequenz infolge des erhöhten Widerstandes kommen.

Ein Hautbereich, in welchem dem Aufstechen eine erhöhte Widerstandskraft entgegensetzt wird, wird als "härtere Haut" bezeichnet. Beim Übergang in einen solchen Hautbereich kann es zum ungleichmäßigen Farbeintrag für das permanente Make-up oder das Tattoo kommen.

In dem Dokument US-A-5 976 167 wird eine Nadelvorrichtung zur Behandlung chronischer Muskelschmerzen beschrieben. Die Nadelvorrichtung umfaßt eine Nadel zur intramuskulären Stimulierung sowie ein Schubwerkzeug zum Aufnehmen der Nadel. Mit Hilfe eines Motors wird die Nadel mittels des Schubwerkzeuges in eine repetierende Schubbewegung versetzt.

Das Dokument US-A-5 054 339 befaßt sich mit der Zuführung von Tätowierfarbstoff von einem Behälter zu einer Tätowiernadel. Mittels einer Pumpe wird die Zuführgeschwindigkeit des Farbstoffes zu der Nadel gesteuert.

Die Dokumente US 4,782,725 und US 4,671,277 beschreiben jeweils eine Tätowiervorrichtung, bei der die Geschwindigkeit und / oder die Repetierfrequenz der Schubbewegung der Tätowiernadel gesteuert wird. Die Steuerung erfolgt manuell.

Schließlich ist bei einer in dem Dokument US 5,471,102 beschriebenen Tätowiervorrichtung die Repetierfrequenz aufgrund von Schaltungsparametern einer die Nadelbewegung steuernden Schaltung festgelegt.

### Die Erfindung

Aufgabe der Erfindung ist es, eine Antriebsvorrichtung für ein Gerät zum lokalen Aufstechen einer Haut, insbesondere zum Aufbringen von permanentem Make-up oder eines Tattoos, und ein Verfahren zum Betreiben der Antriebsvorrichtung anzugeben, die eine individuelle Anpassung an die unterschiedlichen und sich während eines Vorgangs zum lokalen Aufstechen der Haut gegebenenfalls mehrfach ändernden Anwendungsbedingungen ermöglichen.

Diese Aufgabe wird erfindungsgemäß durch eine Antriebsvorrichtung nach dem unabhängigen Anspruch 1 sowie ein Verfahren nach dem unabhängigen Anspruch 8 gelöst.

Nach einem Aspekt der Erfindung ist eine Antriebsvorrichtung für ein Gerät zum lokalen Aufstechen einer Haut, insbesondere zum Aufbringen von permanentem Make-up oder eines Tattoos, mit Antriebsmitteln zum Erzeugen einer repetierenden Schubbewegung geschaffen, die zum Ein- / Ausfahren von Aufstechmitteln nutzbar ist, wobei die Antriebsvorrichtung die folgenden Merkmale aufweist: eine Erfassungseinrichtung, mit der eine Kenngröße erfaßbar ist, die eine der repetierenden Schubbewegung entgegengesetzten Widerstandskraft anzeigt; eine Einstelleinrichtung, mit der für den Fall, daß mit der Erfassungseinrichtung eine geänderte Widerstandskraft erfaßt wird, durch einen Nutzer für die Antriebsmittel ein BetriebsartWechsel in eine Betriebsart erhöhter Repetierfrequenz, in welcher die Repetierfrequenz der repetierenden Schubbewegung gegenüber der Repetierfrequenz vor dem Erfassen der geänderten Widerstandskraft erhöht ist, oder ein Betriebsartwechsel in eine Betriebsart verminderter Repetierfrequenz voreinstellbar sind, in welcher die Repetierfrequenz der repetierenden Schubbewegung gegenüber der Repetierfrequenz vor dem Erfassen der geänderten Widerstandskraft vermindert ist; und eine an die Erfassungseinrichtung und die Einstelleinrichtung gekoppelte Steuereinrichtung, mit der der Betriebsartwechsel in die Betriebsart erhöhter Repetierfrequenz oder in die Betriebsart verminderter Repetierfrequenz einer Voreinstellung der Einstelleinrichtung entsprechend gesteuert wird.

Nach einem weiteren Aspekt der Erfindung ist ein Verfahren zum Betreiben einer Antriebsvorrichtung für ein Gerät zum lokalen Aufstechen einer Haut geschaffen, insbesondere zum Aufbringen von permanentem Make-up oder eines Tattoos, wobei die Antriebsvorrichtung Antriebsmittel zum Erzeugen einer repetierenden Schubbewegung zum Ein- / Ausfahren von Aufstechmitteln umfaßt und wobei das Verfahren die folgenden Schritte aufweist: Erfassen einer Kenngröße mit einer Erfassungseinrichtung, die eine der repetierenden Schubbewegung entgegengesetzte Widerstandskraft beim Ausfahren anzeigt; und Steuern eines Betriebsartwechsels mit Hilfe einer an die Erfassungseinrichtung und die Antriebsmittel gekoppelten Steuereinrichtung in eine Betriebsart erhöhter Repetierfrequenz oder eine Betriebsart verminderter Repetierfrequenz einer Voreinstellung einer Einstelleinrichtung entsprechend; wobei die Einstelleinrichtung für den Fall, daß mit der Erfassungseinrichtung eine geänderte Widerstandskraft erfaßt wird, durch einen Nutzer voreingestellt ist, so daß der Betriebsartwechsel wahlweise in die Betriebsart erhöhter Repetierfrequenz, in welcher die Repetierfrequenz der repetierenden Schubbewegung gegenüber der Repetierfrequenz vor dem Erfassen der geänderten Widerstandskraft erhöht ist, oder in die Betriebsart verminderter Repetierfrequenz festgelegt ist, in welcher die Repetierfrequenz der repetierenden Schubbewegung gegenüber der Repetierfrequenz vor dem Erfassen der geänderten Widerstandskraft vermindert ist.

Die Erfindung betrifft Ausgestaltungen, bei denen mit Hilfe der Einstelleinrichtung durch den Nutzer nur die Voreinstellung des Wechsel in die Betriebsart erhöhter Repetierfrequenz / verminderter Repetierfrequenz ermöglicht ist, was bedeutet, daß die Einstelleinrichtung für den Fall erhöhter Widerstandskraft nur die Wahl zwischen Betriebsartwechsel oder keinem Betriebsartwechsel erlaubt. Von der Erfindung sind jedoch auch Ausführungen umfaßt, bei denen der Nutzer für den Fall erhöhter Widerstandskraft mit Hilfe der Einstelleinrichtung zwischen zwei Arten des Betriebsartwechsels wählen kann, nämlich einem Wechsel in die Betriebsart erhöhter Repetierfrequenz und einem Wechsel in die Betriebsart verminderter Repetierfrequenz, sofern von dem Nutzer eine Voreinstelllung vorgenommen wird. Die Betriebsarten erhöhter / verminderter Repetierfrequenz können jeweils mehrstufig ausgestaltet sein, so daß mehrere Betriebsarten erhöhter Repetierfrequenz und / oder mehrere Betriebsarten verminderter Repetierfrequenz zur Verfügung stehen, was mit Hilfe eines geeigneten Wahlschalters erfolgen kann.

Beim Ein- / Ausfahren der Aufstechmittel wird eine Spitze der Aufstechmittel beim Einfahren entweder durch eine Öffnung in das Gehäuse zurück gezogen oder verbleibt vor der Gehäuseöffung außerhalb des Gehäuses. Auf diese Weise ist insbesondere eine Betriebsart realisierbar, bei der zumindest die Spitze der Aufstechmittel während des Betriebs ständig außerhalb des Gehäuses des Handgerätes verbleibt, auch wenn ein Vor- / Zurückbewegen der Aufstechmittel repetierend ausgeführt wird.

Die Elemente der Antriebsvorrichtung können sowohl in einem zum Führen der Nadel gebildeten Handgerät als auch in einem zum Steuern des Handgerätes üblicherweise vorgesehenen Steuergerät gebildet sein. Handgerät und Steuergerät sind über einer Steuerkabel verbunden, welches in der Regel auch eine Spannungsversorgungsleitung umfaßt, um eine in dem Steuergerät gebildet Betriebsspannung an den Antriebsmitteln zur Verfügung zu stellen. Im Handgerät befinden sich die Antriebsmittel, welche insbesondere einen Motor umfassen können. Andere Elemente der Antriebsvorrichtung wie die Steuereinrichtung können in dem Handgerät oder dem Steuergerät angeordnet werden. Die Erfassungseinrichtung ist in dem Handgerät oder dem Steuergerät gebildet, was insbesondere davon abhängt, welche Kenngröße erfaßt wird. Wenn eine Drehzahl am Motor zu messen ist, kann die Erfassungseinrichtung in dem Handgerät angeordnet sein. Wenn jedoch bei einer möglichen Ausführung die Stromaufnahme als Kenngröße ausgewertet wird, so kann die Erfassung dieses Meßwertes in dem Steuergerät ausgeführt werden. Die Einstelleinrichtung und die Steuereinrichtung können wahlweise jeweils in das Steuergerät oder das Handgerät integriert sein.

Ein wesentlicher Vorteil, welcher mit der Erfindung gegenüber dem Stand der Technik erreicht wird, besteht darin, daß dem Nutzer der Antriebsvorrichtung für das Gerät zum lokalen Aufstechen der Haut die Möglichkeit zur Verfügung gestellt ist, auf individuelle Anforderungen beim Behandeln der Haut einzugehen, indem für den Fall geänderter Hauteigenschaften vorab ein automatischer Betriebsartwechsel einstellbar ist, so daß die Repetierfrequenz der repetierenden Schubbewegung beim Aufstechen der Haut erhöht / vermindert wird. Die Voreinstellung kann durch den Nutzer den unterschiedlichen Anforderungen und seinen Wünschen entsprechend erfolgen. Auch das Einbringen eines kosmetischen Wirkstoffe über die Haut mit Hilfe des lokalen Aufstechens kann vorgesehen sein. Auf diese Weise wird die Bedienfreundlichkeit der Geräte verbessert. Die das Gerät bedienende Person kann sich ganz auf das Aufstechen der Haut konzentrieren, da beim Auftreten einer geänderter Widerstandskraft, d. h. einer geänderten Hautoberfläche, das Umschalten in die Betriebsart erhöhter / verminderter Repetierfrequenz der Voreinstellung entsprechend automatisch erfolgt, ohne daß ein zusätzlicher Eingriff durch das Bedienpersonal notwendig ist. Ein Betriebsartwechsel kann mehrfach während der Nutzung des Gerätes stattfinden, wenn mehrfach zwischen verschiedenen Hautpartien gewechselt wird.

Wenn ein Übergang zu härterer Haut erfolgt, kann mit Hilfe der Erfindung verhindert werden, daß die Nadel stecken bleibt, was mittels des automatischen Überganges zu einer Betriebsart veränderter Frequenz erreicht wird.

Gesteuert durch die Steuereinrichtung erfolgt der Betriebsartwechsel bei verschiedenen Ausführungsformen der Erfindung der Voreinstellung entsprechend entweder automatisch oder nachdem die Steuereinrichtung eine Bestätigung des Betriebsartwechsels durch den Nutzer, beispielsweise indem ein Tastsignal abgewartet wird, erfaßt hat. Im letzten Fall wird mit Hilfe der Steuereinrichtung bevorzugt ein optisches oder akustisches Signal erzeugt, welches dem Nutzer den Betriebsartwechsel anzeigt, so daß der Nutzer diesen bestätigen kann.

Bei einer zweckmäßigen Ausgestaltung der Erfindung ist vorgesehen, daß die Erfassungseinrichtung konfiguriert ist, um als Kenngröße eine Stromaufnahme der Antriebsmittel zu erfassen. Wenn sich die der repetierenden Schubbewegung entgegengesetzte Widerstandskraft beim Ausfahren vermindert oder erhöht, so führt dies bei den Antriebsmitteln zu einer Änderung der Stromaufnahme, weshalb diese Kenngröße ein zuverlässiger Indikator für die Änderung der Widerstandskraft ist.

Eine mit Hilfe üblicher Sensoren exakt erfaßbare Meßgröße ist die Drehzahl von Bau- oder Maschinenteilen, weshalb bei einer bevorzugten Ausführungsform der Erfindung vorgesehen ist, daß die Erfassungseinrichtung konfiguriert ist, um als Kenngröße eine Drehzahl der Antriebsmittel zu erfassen. Als Antriebsmittel werden häufig Motoren verwendet, die eine Drehbewegung erzeugen, welche dann mit Hilfe von Kopplungs- oder Getriebemitteln in eine lineare repetierende Bewegung zum Ein- / Ausfahren der Nadel oder des Nadelsystems in dem Gerät zum lokalen Aufstechen der Haut umgesetzt wird.

Bei der Nutzung eines Drehmotors als Antriebsmittel sieht eine vorteilhafte Weiterbildung der Erfindung vor, daß die Erfassungseinrichtung konfiguriert ist, um als Kenngröße eine Kommutationsfrequenz an dem Drehmotor zu erfassen. Die Kommutationsfrequenz ist ihrerseits proportional zur Drehzahl des Drehmotors, die sich bei einer Änderung der Widerstandskraft ändert.

Es kann auch vorgesehen sein, die Häufigkeit der Hübe der Schubbewegung zum Ausfahren der Nadel als Kenngröße zu erfassen.

Um die Bedingungen beim lokalen Aufstechen, insbesondere zum Aufbringen des permanentem Make-ups oder des Tattoos, möglichst kontinuierlich reproduzierbar zu halten, sieht eine Weiterbildung der Erfindung vor, daß die Antriebsmittel konfiguriert sind, um die Repederfrequenz vor dem Erfassen der geänderten Widerstandskraft, in der Betriebsart erhöhter Repetierfrequenz und in der Betriebsart verminderter Repetierfrequenz im wesentlichen konstant zu halten. Eine konstante Repetierfrequenz wird mittels Nachregeln der elektrischen Kenngröße(n) der Antriebsmittel erreicht, beispielsweise der Stromaufnahme und / oder der angelegten Spannung an dem Antriebsmotor.

Es besteht die Möglichkeit, die erfaßte Kenngröße nicht nur qualitativ, d. h. hinsichtlich einer Änderung, sondern auch quantitativ auszuwerten mit Hilfe der Steuermittel. In diesem Zusammenhang sieht eine zweckmäßige Ausführungsform der Erfindung vor, daß die Steuereinrichtung konfiguriert sind, um bei dem Betriebsartwechsel die erhöhte / verminderte Repetierfrequenz in der Betriebsart erhöhter Repetierfrequenz / verminderter Repetierfrequenz in Abhängigkeit von einem oder mehreren Meßwerten für die erfaßte Kenngröße festzulegen. In Abhängigkeit von einer quantitativen Auswertung der erfaßten Kenngröße kann dann die Repetierfrequenz für das Betreiben des Gerätes nach dem Betriebsartwechsel festgelegt werden. Beispielsweise können bestimmte Schwellwerte für die Kenngröße festgelegt werden, für dann jeweils eine zugehörige einzustellende Repetierfrequenz bestimmt ist. Auf diese Weise ist eine noch weitergehende Individualisierung der Betriebsart in Abhängigkeit von den Anwendungsbedingungen ermöglicht, insbesondere der Hauteigenschaften.

Bevorzugt kann bei einer Ausgestaltung der Erfindung vorgesehen sein, daß die Steuereinrichtung konfiguriert ist, um den Betriebsartwechsel in die mittels der Einstelleinrichtung voreingestellte Betriebsart erhöhter Repetierfrequenz / verminderter Repetierfrequenz auszulösen, wenn die erfaßte Kenngröße eine Erhöhung der Widerstandskraft anzeigt. Auf diese Weise ist es ermöglicht, beim Übergang zu "härterer Haut" automatisch einen Betriebsartwechsel zu vollziehen. Insbesondere bei einem solchen Übergang ist es wichtig, eine Anpassung an die geänderten Eigenschaften der Hautoberfläche zu vollziehen, da sich die Bedingungen für das Aufstechen der Haut ändern. Darüber hinaus erfordert diese Situation die besondere Aufmerksamkeit und die Konzentration des Bedienpersonals auf das korrekte Aufstechen der Haut, ohne daß zusätzlich noch die Repetierfrequenz beobachtet werden kann.

In Verbindung mit den abhängigen Ansprüchen des Verfahrens zum Betreiben einer Antriebsvorrichtung für ein Gerät zum lokalen Aufstechen einer Haut, insbesondere zum Aufbringen von permanentem Make-up oder eines Tattoos, gelten die in Verbindung mit den zugehörigen Ansprüchen zur Antriebsvorrichtung gemachten Ausführungen zu Vorteilen entsprechend.

### Bevorzugte Ausführunsgbeispiele der Erfindung

Die Erfindung wird im folgenden unter Bezugnahme auf die Figuren einer Zeichnung näher erläutert. Hierbei zeigen:
- Fig. 1: eine schematische Schnittdarstellung eines Geräts zum lokalen Aufstechen einer Haut, insbesondere zum Aufbringen von permanentem Make-up oder eines Tattoos, mit einer Antriebsvorrichtung und einem hieran gekoppelten Modul mit Aufstechmitteln; und
- Fig. 2: eine schematische Blockdarstellung von Elementen der Antriebsvorrichtung.

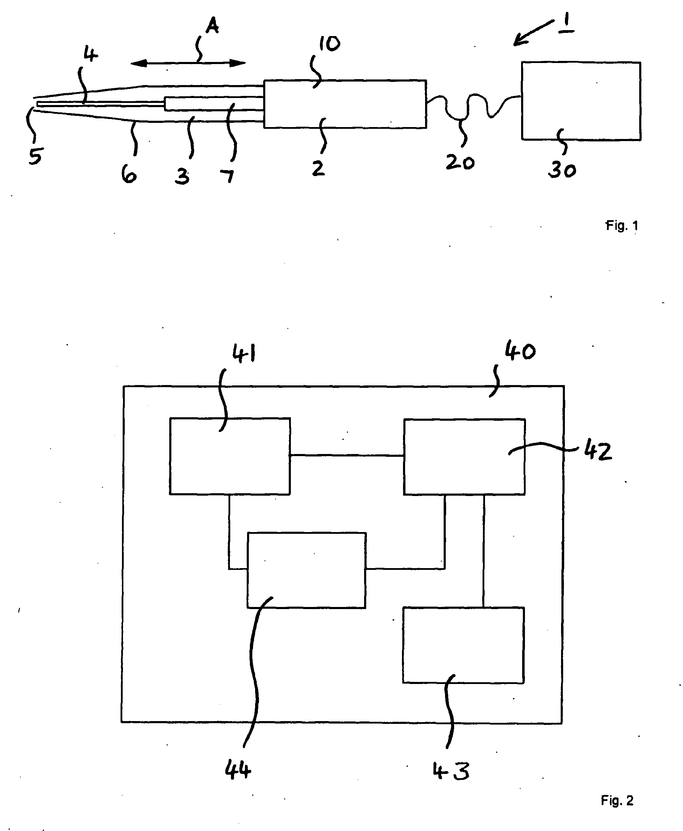

Fig. 1 zeigt schematisch eine Schnittdarstellung eines Gerätes 1 zum lokalen Aufstechen einer Haut, insbesondere zum Aufbringen von permanentem Make-up oder eines Tattoos oder zum Einbringen eines medizinischen oder eines kosmetischen Wirkstoffes, mit einem Handgerät 10 und einem an das Handgerät über ein Kabel 20 angeschlossenes Steuergerät 30. An dem Handgerät 10 sind ein Antriebsmodul 2 und ein hieran gekoppeltes Modul 3. Das Modul 3 kann lösbar oder fest mit dem Antriebsmodul 2 verbunden sein. Eine lösbare Ausführung hat den Vorteil, daß das Modul 3 mit verschiedenen Antriebsmodulen nutzbar ist und auch als Einwegmodul ausführbar ist.

Mit Hilfe des Antriebsmoduls 2 wird eine Antriebskraft zur Verfügung gestellt, beispielsweise mittels eines Drehmotors oder eines über Magnetspulen angetriebenen Motors. Derartige Einrichtungen zum Erzeugen einer Antriebskraft sind in verschiedenen Ausführungen bekannt und werden hier deshalb nicht näher erläutert.

Die erzeugte Antriebskraft wird in Verbindung mit einer Dreh-, einer Kipp-, einer Linearbewegung oder dergleichen zur Verfügung gestellt. Die Antriebskraft wird in eine repetierende Schubbewegung eingeleitet, die in Fig. 1 mit Hilfe eines Pfeiles A dargestellt ist. Infolge der repetierenden Schubbewegung wird eine Nadel 4, bei der es sich um eine Einzelnadel oder ein Nadelsystem mit mehreren Nadeln handeln kann, durch eine Öffnung 5 in einem Gehäuse 6 aus- und eingefahren. Die Nadel 4 ist in einem Nadelschaft 7 gelagert. Beim Ausfahren der Nadel 4 wird die zu behandelnde Haut aufgestochen, so daß beispielsweise die Farbe zum Ausbilden des permanentem Make-ups oder des Tattoos in die Hautoberfläche eindringen kann. Beim Aufstechen der Haut 4 wird der Nadel durch die Hautoberfläche eine von deren Eigenschaften abhängige Widerstandskraft entgegengesetzt.

Die Nadel 4 kann direkt oder über den Nadelschaft 7 an Kopplungs- / Getriebemittel (nicht dargestellt) des Antriebsmoduls 2 gekoppelt sein, so daß die Rückführung der Nadel 4 nach dem Aufstechen der Haut mittels der Rückbewegung der Kopplungs- / Getriebemittel erfolgt. Alternativ kann vorgesehen sein, daß die Nadel 4 mit Hilfe von Rückstellmitteln rückgeführt wird.

Es ist eine Antriebsvorrichtung 40 mit Elementen vorgesehen, mit denen die Ausgestaltung der repetierenden Schubbewegung in Abhängigkeit von Betriebsbedingungen automatisch einstellbar ist und die in Fig. 2 mit Hilfe einer schematischen Blockdarstellung gezeigt sind. Die Elemente der Antriebsvorrichtung 40 können in dem Handgerät 10 und / oder in dem Steuergerät 30 gebildet sein. Mit Hilfe einer Erfassungseinrichtung 40 werden Meßwerte für eine Kenngröße erfaßt, welche die der Nadel 4 (vgl. Fig. 1) entgegengesetzte Widerstandskraft der Hautoberfläche charakterisiert. Hierbei handelt es sich beispielsweise um die Drehzahl eines Bau- oder Maschinenteils von Antriebsmitteln 41 oder die Strom- / Spannungsaufnahme der Antriebsmittel 41. Beide Werte ändern sich, wenn die Widerstandskraft auf die Nadel 4 zu- oder abnimmt.

Wenn eine solche Änderung der Widerstandskraft ermittelt wird, veranlaßt eine Steuereinrichtung 42, die an die Antriebsmittel 41 und die Erfassungseinrichtung 44 gekoppelt ist, einen Betriebsartwechsel der Antriebsmittel 41, so daß mit einer Betriebsart erhöhter Repetierfrequenz der repetierenden Schubbewegung oder mit einer Betriebsart verminderter Repetierfrequenz der repetierenden Schubbewegung fortgesetzt wird. Die Repetierfrequenz wird erhöht / vermindert im Vergleich zu einer Repetierfrequenz vor dem Erfassen der Änderung der Widerstandskraft.

In welche der geänderten Betriebsarten gewechselt wird, erfolgt in Abhängigkeit von einem Einstellzustand einer Einstelleinrichtung 43, die mit der Steuereinrichtung 42 verbunden ist. Die Einstelleinrichtung 43 ist beispielsweise als Tast-, Schiebe- oder Drehschalter, verbunden gegebenenfalls mit einer Anzeigeeinrichtung, ausgeführt, so daß das Bedienpersonal mit Hilfe der Einstelleinrichtung 43 festlegen kann, in welche Betriebsart automatisch gewechselt wird, wenn eine Änderung der Widerstandskraft auf die repetierende Schubbewegung festgestellt wird. Beispielsweise kann vorgesehen sein, daß in einer normalen oder anfänglichen Betriebsart eine Repetierfrequenz von 120Hz eingestellt ist. Wenn eine Änderung der Widerstandskraft erfaßt wird, soll im Fall des Wechsels in die Betriebsart mit erhöhter Repetierfrequenz mit einer Frequenz von 130Hz und im Fall des Wechsels in die Betriebsart mit verminderter Repetierfrequenz mit einer Repetierfrequenz von 110Hz fortgefahren werden. Ein solcher Betriebsartwechsel soll insbesondere dann stattfinden, wenn eine Erhöhung der Widerstandskraft festgestellt wird, was den Übergang in einen Hautbereich anzeigt, der härter ist.

Mit Hilfe der Einstelleinrichtung 43 können auch jeweils mindestens zwei Betriebsarten mit erhöhter Repetierfrequenz / verminderter Repetierfrequenz für eine Benutzerauswahl zu Verfügung gestellt werden. Entsprechende Wahlschalter stehen in unterschiedlichen Ausführungen zur Verfügung. In einer besonders einfachen Ausführung ist mit der Einstelleinrichtung 43 dem Nutzer lediglich die Möglichkeit gegeben, einen bestimmten Betriebsartwechsel voreinzustellen oder auf diesen zu verzichten.

Vorzugsweise ist die Antriebsvorrichtung 40, insbesondere unter Verwendung der in Fig. 2 dargestellten Komponenten, so konfiguriert, daß die Repetierfrequenz in den verschiedenen Betriebsarten im wesentlichen konstant gehalten wird. Dieses erfolgt zweckmäßig über eine Nachregelung der Strom- und / oder der Spannungsaufnahme der Antriebsmittel 41.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Antriebsvorrichtung (40) zum lokalen Aufstechen einer Haut, insbesondere zum Aufbringen von permanentem Make-up oder eines Tattoos, mit Antriebsmitteln (41) zum Erzeugen einer repetierenden Schubbewegung (A), die zum Ein- / Ausfahren von Aufstechmitteln (4) nutzbar ist, **gekennzeichnet durch**
- eine Erfassungseinrichtung (44), mit der eine Kenngröße erfaßbar ist, die eine der repetierenden Schubbewegung entgegengesetzten Widerstandskraft anzeigt;
- eine Einstelleinrichtung (43), mit der für den Fall, daß mit der Erfassungseinrichtung (44) eine geänderte Widerstandskraft erfaßt wird, **durch** einen Nutzer für die Antriebsmittel (41) ein Betriebsartwechsel in eine Betriebsart erhöhter Repetierfrequenz, in welcher die Repetierfrequenz der repetierenden Schubbewegung (A) gegenüber der Repetierfrequenz vor dem Erfassen der geänderten Widerstandskraft erhöht ist, oder ein Betriebsartwechsel in eine Betriebsart verminderter Repetierfrequenz voreinstellbar sind, in welcher die Repetierfrequenz der repetierenden Schubbewegung (A) gegenüber der Repetierfrequenz vor dem Erfassen der geänderten Widerstandskraft vermindert ist; und
- eine an die Erfassungseinrichtung (44) und die Einstelleinrichtung (43) gekoppelte Steuereinrichtung (42), mit der der Betriebsartwechsel in die Betriebsart erhöhter Repetierfrequenz oder in die Betriebsart verminderter Repetierfrequenz einer Voreinstellung der Einstelleinrichtung (43) entsprechend gesteuert wird.

2. Antriebsvorrichtung nach Anspruch 1, **dadurch gekennzeichnet, dass** die Erfassungseinrichtung (44) konfiguriert ist, um als Kenngröße ein Stromaufnahme der Antriebsmittel (41) zu erfassen.

3. Antriebsvorrichtung nach Anspruch 1 oder 2, **dadurch gekennzeichnet, dass** die Erfassungseinrichtung (44) konfiguriert ist, um als Kenngröße eine Drehzahl der Antriebsmittel (41) zu erfassen.

4. Antriebsvorrichtung nach einem der vorangehenden Ansprüche, **dadurch gekennzeichnet, dass** die Antriebsmittel (41) einen Bürstenmotor umfassen und die Erfassungseinrichtung (44) konfiguriert ist, um als Kenngröße eine Kommutationsfrequenz des Bürstenmotors zu erfassen.

5. Antriebsvorrichtung nach einem der vorangehenden Ansprüche, **dadurch gekennzeichnet, dass** die Antriebsmittel (41) konfiguriert sind, um die Repetierfrequenz vor dem Erfassen der geänderten Widerstandskraft, in der Betriebsart erhöhter Repetierfrequenz und in der Betriebsart verminderter Repetierfrequenz im wesentlichen konstant zu halten.

6. Antriebsvorrichtung nach einem der vorangehenden Ansprüche, **dadurch gekennzeichnet, dass** die Steuereinrichtung (42) konfiguriert ist, um bei dem Betriebsartwechsel die erhöhte / verminderte Repetierfrequenz in der Betriebsart erhöhter Repetierfrequenz / verminderter Repetierfrequenz in Abhängigkeit von einem oder mehreren Meßwerten für die erfaßte Kenngröße festzulegen.

7. Antriebsvorrichtung nach einem der vorangehenden Ansprüche, **dadurch gekennzeichnet, dass** die Steuereinrichtung (42) konfiguriert ist, um den Betriebsartwechsel in die mittels der Einstelleinrichtung voreingestellte Betriebsart erhöhter Repetierfrequenz / verminderter Repetierfrequenz auszulösen, wenn die erfaßte Kenngröße eine Erhöhung der Widerstandskraft anzeigt.

8. Verfahren zum Betreiben einer Antriebsvorrichtung (40) für ein Gerät zum lokalen Aufstechen einer Haut, insbesondere zum Aufbringen von permanentem Make-up oder eines Tattoos, wobei die Antriebsvorrichtung (40) Antriebsmittel (41) zum Erzeugen einer repetierenden Schubbewegung (A) aufweist, die zum Ein-/Ausfahren von Aufstechmitteln (4) nutzbar ist, und wobei das Verfahren die folgenden Schritte umfaßt:
- Erfassen einer Kenngröße mit einer Erfassungseinrichtung (44), die eine der repetierenden Schubbewegung (A) entgegengesetzte Widerstandskraft anzeigt; und
- Steuern eines Betriebsartwechsels mit Hilfe einer an die Erfassungseinrichtung (44) und die Antriebsmittel (41) gekoppelten Steuereinrichtung (42) in eine Betriebsart erhöhter Repetierfrequenz oder eine Betriebsart verminderter Repetierfrequenz einer Voreinstellung einer Einstelleinrichtung (43) entsprechend;
wobei die Einstelleinrichtung (43) für den Fall, daß mit der Erfassungseinrichtung (44) eine geänderte Widerstandskraft erfaßt wird, durch einen Nutzer voreingestellt ist, so daß der Betriebsartwechsel wahlweise in die Betriebsart erhöhter Repetierfrequenz, in welcher die Repetierfrequenz der repetierenden Schubbewegung (A) gegenüber der Repetierfrequenz vor dem Erfassen der geänderten Widerstandskraft erhöht ist, oder in die Betriebsart verminderter Repetierfrequenz festgelegt ist, in welcher die Repetierfrequenz der repetierenden Schubbewegung (A) gegenüber der Repetierfrequenz vor dem Erfassen der geänderten Widerstandskraft vermindert ist.

9. Verfahren nach Anspruch 8, **dadurch gekennzeichnet, daß** mit der Erfassungseinrichtung (44) als Kenngröße eine Stromaufnahme der Antriebsmittel erfaßt wird.

10. Verfahren nach Anspruch 8 oder 9, **dadurch gekennzeichnet, daß** mit der Erfassungseinrichtung (44) als Kenngröße eine Drehzahl der Antriebsmittel (41) erfaßt wird.

11. Verfahren nach einem der Ansprüche 8 bis 10, **dadurch gekennzeichnet, daß** die Antriebsmittel (41) einen Drehmotor umfassen und mit der Erfassungseinrichtung (44) als Kenngröße eine Kommutationsfrequenz des Drehmotors erfaßt wird.

12. Verfahren nach einem der Ansprüche 8 bis 11, **dadurch gekennzeichnet, daß** die Repetierfrequenz vor dem Erfassen der geänderten Widerstandskraft, in der Betriebsart erhöhter Repetierfrequenz und in der Betriebsart verminderter Repetierfrequenz im wesentlichen konstant gehalten wird.

13. Verfahren nach einem der Ansprüche 8 bis 12, **dadurch gekennzeichnet, daß** mit der Steuereinrichtung (43) bei dem Betriebsartwechsel die erhöhte / verminderte Repetierfrequenz in der Betriebsart erhöhter Repetierfrequenz / verminderter Repetierfrequenz in Abhängigkeit von einem oder mehreren Meßwerten für die erfaßte Kenngröße festgelegt wird.

14. Verfahren nach einem der Ansprüche 8 bis 13, **dadurch gekennzeichnet, daß** die Steuereinrichtung (42) den Betriebsartwechsel in die mittels der Einstelleinrichtung (43) voreingestellte Betriebsart erhöhter Repetierfrequenz / verminderter Repetierfrequenz auslöst, wenn die erfaßte Kenngröße ab Erhöhung der Widerstandskraft anzeigt.

15. Verfahren nach einem der Ansprüche 8 bis 14, **dadurch gekennzeichnet, daß** mit Hilfe der Steuereinrichtung (42) vor dem Betriebsartwechsel eine Bestätigung durch einen Nutzer erfaßt wird.

## Claims

1. Driving device (40) for the local puncturing of a skin, particularly for the application of permanent make-up or a tattoo, with drive means (41) for producing a repetitive thrust movement (A) which is usable for the retraction / extension of puncturing means (4), **characterized by**
- a detection apparatus (44) with which a parameter is detectable that indicates a resistance force acting against the repetitive thrust movement;
- a setting apparatus (43) with which, for the case that a changed resistance force is detected with the detection apparatus (44), by a user for the drive means (41), an operating mode change into an operating mode of increased repeating frequency in which the repeating frequency of the repetitive thrust movement (A) compared with the repeating frequency is increased before the detection of the changed resistance force, or an operating mode change into an operating mode of reduced repeating frequency can be pre-set in which the repeating frequency of the repetitive thrust movement (A) compared with the repeating frequency is decreased before detection of the change resistance force; and
- a control apparatus (42) coupled to the detection apparatus (44) and the setting apparatus (43), with which the operating mode change into the operating mode of increased repeating frequency or into the operating mode of reduced repeating frequency is controlled corresponding to a pre-setting of the setting apparatus (43).

2. Driving device according to Claim 1, **characterized in that** the detection apparatus (44) is configured in order to detect as a parameter a power input of the drive means (41).

3. Driving device according to Claim 1 or 2, **characterized in that** the detection apparatus (44) is configured in order to detect as a parameter a speed of the drive means (41).

4. Driving device according to any one of the preceding Claims, **characterized in that** the drive means (41) comprise a brush motor, and the detection apparatus (44) is configured in order to detect as a parameter a commutation frequency of the brush motor.

5. Driving device according to any one of the preceding Claims, **characterized in that** the drive means (41) are configured in order to maintain at an essentially constant level the repeating frequency before the detection of the changed resistance force, in the operating mode of increased repeating frequency and in the operating mode reduced repeating frequency.

6. Driving device according to any one of the preceding Claims, **characterized in that** the control apparatus (42) is configured in order to stipulate, during the operating mode change, the increased / reduced repeating frequency in the operating mode of increased repeating frequency / reduced repeating frequency in dependence of one or several measuring values for the detected parameter.

7. Driving device according to any one of the preceding Claims, **characterized in that** the control apparatus (42) is configured in order to change the operating mode into the operating mode, pre-set by means of the setting apparatus, of increased repeating frequency / reduced repeating frequency if and when the detected parameter indicates an increase of the resistance force.

8. Method for the operation of a driving device (40) for a device for the local puncturing of a skin, particularly for the application of permanent make-up or a tattoo, where the driving device (40) has drive means (41) for producing a repetitive thrust movement (A) that is usable for the retraction / extension of puncturing means (4), and where the method comprises the following steps:
- Detection of a parameter with a detection apparatus (44), which indicates a resistance force acting against the repetitive thrust movement (A); and
- Control of an operating mode change with the help of a control apparatus (42) coupled to the detection apparatus (44) and the drive means (41) into an operating mode of increased repeating frequency or an operating mode of reduced repeating frequency in accordance with a pre-setting of a setting apparatus (43);
where the setting apparatus (43) for the case that a changed resistance force is detected with the detection apparatus (44), is pre-set by a user so that the operating mode change is selectively stipulated in the operating mode of increased repeating frequency, in which the repeating frequency of the repetitive thrust movement (A) is increased compared with the repeating frequency before detection of the changed resistance force, or in the operating mode of reduced repeating frequency in which the repeating frequency of the repetitive thrust movement (A) is reduced compared with the repeating frequency before the detection of the change resistance force.

9. Method according to Claim 8, **characterized in that**, with the detection apparatus (44), a power input of the drive means (41) is detected as a parameter.

10. Method according to Claim 8 or 9, **characterized in that**, with the detection apparatus (44), a rotational speed of the drive means (41) is detected as a parameter.

11. Method according to any one of the Claims 8 to 10, **characterized in that** the drive means (41) comprise a rotating motor and that a commutation frequency of the rotating motor is detected as a parameter using the detection apparatus (44).

12. Method according to any one of the Claims 8 to 11, **characterized in that** the repeating frequency, before detection of the changed resistance force, is essentially kept at a constant level in the operating mode of increased repeating frequency and in the operating mode of reduced repeating frequency.

13. Method according to any one of the Claims 8 to 12, **characterized in that**, with the control apparatus (43) during operating mode change, the increased / reduced repeating frequency is stipulated in the operating mode of increased repeating frequency / reduced repeating frequency in dependence of one or several measuring values for the detected parameter.

14. Method according to any one of the Claims 8 to 13, **characterized in that** the control apparatus (42) changes the operating mode into the operating mode, pre-set with the setting apparatus (43), of increased repeating frequency / reduced repeating frequency if and when the detected parameter is indicated as from increase of the resistance force.

15. Method according to any one of the Claims 8 to 14, **characterized in that**, with the help of the control apparatus (42) an acknowledgement by a user is detected before the operating mode change.

## Revendications

1. Dispositif d'entraînement (40) pour le perçage local d'une peau, en particulier pour l'application de fond de teint permanent ou d'un tatouage, avec des moyens d'entraînement (41) pour la génération d'un mouvement de translation répétitif (A) qui peut être utilisé pour la rétraction / le déploiement de moyens de perçage (4), **caractérisé par**
- un équipement de saisie (44) qui permet de saisir une grandeur caractéristique qui indique une force de résistance opposée au mouvement de translation répétitif ;
- un équipement de réglage (43) avec lequel, dans le cas où une modification de la force de résistance est saisie avec l'équipement de saisie (44), un utilisateur peut prérégler pour les moyens d'entraînement (41) un changement de mode de fonctionnement pour ajuster un mode de fonctionnement à fréquence de répétition accrue dans lequel la fréquence de répétition du mouvement de translation répétitif (A) est accrue par rapport à la fréquence de répétition avant la saisie de la force de résistance modifiée, ou un changement de mode de fonctionnement pour ajuster un mode de fonctionnement à fréquence de répétition réduite dans lequel la fréquence de répétition du mouvement de translation répétitif (A) est réduite par rapport à la fréquence de répétition avant la saisie de la force de résistance modifiée ; et
- un équipement de commande (42) accouplé à l'équipement de saisie (44) et à l'équipement de réglage (43), qui permet de commander le changement de mode de fonctionnement pour ajuster le mode de fonctionnement à fréquence de répétition accrue ou le mode de fonctionnement à fréquence de répétition réduite en fonction d'un préréglage de l'équipement de réglage (43).

2. Dispositif d'entraînement selon la revendication 1, **caractérisé en ce que** l'équipement de saisie (44) est configuré de manière à saisir, comme grandeur caractéristique, une consommation de courant des moyens d'entraînement (41).

3. Dispositif d'entraînement selon la revendication 1 ou 2, **caractérisé en ce que** l'équipement de saisie (44) est configuré de manière à saisir, comme grandeur caractéristique, une vitesse de rotation des moyens d'entraînement (41).

4. Dispositif d'entraînement selon l'une quelconque des revendications précédentes, **caractérisé en ce que** les moyens d'entraînement (41) comprennent un moteur à balais et l'équipement de saisie (44) est configuré de manière à saisir, comme grandeur caractéristique, une fréquence de commutation du moteur à balais.

5. Dispositif d'entraînement selon l'une quelconque des revendications précédentes, **caractérisé en ce que** les moyens d'entraînement (41) sont configurés de manière à maintenir essentiellement constante la fréquence de répétition avant la saisie de la force de résistance modifiée dans le mode de fonctionnement à fréquence de répétition accrue et dans le mode de fonctionnement à fréquence de répétition réduite.

6. Dispositif d'entraînement selon l'une quelconque des revendications précédentes, **caractérisé en ce que** l'équipement de commande (42) est configuré de manière à définir, au changement de mode de fonctionnement, la fréquence de répétition accrue / réduite dans le mode de fonctionnement à fréquence de répétition accrue / réduite en fonction d'une ou de plusieurs valeurs de mesure pour la grandeur caractéristique saisie.

7. Dispositif d'entraînement selon l'une quelconque des revendications précédentes, **caractérisé en ce que** l'équipement de commande (42) est configuré de manière à déclencher le changement de mode de fonctionnement pour ajuster le mode de fonctionnement à fréquence de répétition accrue / réduite préréglé au moyen de l'équipement de réglage lorsque la grandeur caractéristique saisie indique un accroissement de la force de résistance.

8. Procédé pour l'exploitation d'un dispositif d'entraînement (40) pour un appareil destiné au perçage local d'une peau, en particulier pour l'application de fond de teint permanent ou d'un tatouage, étant donné que le dispositif d'entraînement (40) présente des moyens d'entraînement (41) pour la génération d'un mouvement de translation répétitif (A) qui peut être utilisé pour la rétraction / le déploiement de moyens de perçage (4), et étant donné que le procédé comprend les étapes suivantes :
- saisie d'une grandeur caractéristique à l'aide d'un équipement de saisie (44) qui indique une force de résistance opposée à un mouvement de translation répétitif (A) ; et
- commande d'un changement de mode de fonctionnement à l'aide d'un équipement de commande (42) accouplé à l'équipement de saisie (44) et aux moyens d'entraînement (41) pour ajuster un mode de fonctionnement à fréquence de répétition accrue ou un mode de fonctionnement à fréquence de répétition réduite en fonction d'un préréglage d'un équipement de réglage (43) ;
étant donné que l'équipement de réglage (43) est préréglé par un utilisateur, pour le cas où une modification de la force de résistance est saisie avec l'équipement de saisie (44), de manière à ce que le changement de mode de fonctionnement s'effectue pour ajuster au choix le mode de fonctionnement à fréquence de répétition accrue dans lequel la fréquence de répétition du mouvement de translation répétitif (A) est accrue par rapport à la fréquence de répétition avant la saisie de la force de résistance modifiée, ou le mode de fonctionnement à fréquence de répétition réduite dans lequel la fréquence de répétition du mouvement de translation répétitif (A) est réduite par rapport à la fréquence de répétition avant la saisie de la force de résistance modifiée.

9. Procédé selon la revendication 8, **caractérisé en ce qu'**une consommation de courant des moyens d'entraînement est saisie comme grandeur caractéristique avec l'équipement de saisie (44).

10. Procédé selon la revendication 8 ou 9, **caractérisé en ce qu'**une vitesse de rotation des moyens d'entraînement (41) est saisie comme grandeur caractéristique avec l'équipement de saisie (44).

11. Procédé selon l'une quelconque des revendications 8 à 10, **caractérisé en ce que** les moyens d'entraînement (41) comprennent un moteur à courant triphasé et **en ce qu'**une fréquence de commutation du moteur à courant triphasé est saisie comme grandeur caractéristique avec l'équipement de saisie (44).

12. Procédé selon l'une quelconque des revendications 8 à 11, **caractérisé en ce que** la fréquence de répétition avant la saisie de la force de répétition modifiée est maintenue essentiellement constante dans le mode de fonctionnement à fréquence de répétition accrue et dans le mode de fonctionnement à fréquence de répétition réduite.

13. Procédé selon l'une quelconque des revendications 8 à 12, **caractérisé en ce que** l'équipement de commande (43) est utilisé pour définir, au changement de mode de fonctionnement, la fréquence de répétition accrue / réduite dans le mode de fonctionnement à fréquence de répétition accrue / réduite en fonction d'une ou de plusieurs valeurs de mesure pour la grandeur caractéristique saisie.

14. Procédé selon l'une quelconque des revendications 8 à 13, **caractérisé en ce que** l'équipement de commande (42) déclenche le changement de mode de fonctionnement pour ajuster le mode de fonctionnement à fréquence de répétition accrue / réduite préréglé au moyen de l'équipement de réglage lorsque la grandeur caractéristique saisie indique un accroissement de la force de résistance.

15. Procédé selon l'une quelconque des revendications 8 à 14, **caractérisé en ce qu'**une confirmation est saisie par un utilisateur à l'aide de l'équipement de commande (42) avant le changement de mode de fonctionnement.
